# FASCICULE DE BREVET EUROPEEN

(11) **EP 1 624 856 B1**
(45) Date de publication et mention de la délivrance du brevet: **14.01.2015**
(21) Numéro de dépôt: 04742674.7
(22) Date de dépôt: 07.05.2004
(51) Int. Cl.: A61K 9/06, A61K 9/50, A61K 31/593

(54) **PROCEDE DE PREPARATION D'UNE POMMADE CONTENANT UN PRINCIPE ACTIF MICRODOSE**
VERFAHREN ZUR HERSTELLUNG EINER FORMULIERUNG MIT EINEM MIKRODOSIERTEN WIRKSTOFF
METHOD OF PREPARING A FORMULATION CONTAINING A MICRODOSED ACTIVE PRINCIPLE

(30) Priorité: 12.05.2003 FR 0305684
(43) Date de publication de la demande: 15.02.2006
(73) Titulaire: Galderma S.A., 6330 Cham (CH)
(72) Inventeur: CUILLERY, Yves, 74330 LA BALME DE SILLIGNY (FR); CHAMBINAUD, Denis, 73000 CHAMBERY (FR)
(74) Mandataire: Tezier Herman, Béatrice
(86) Numéro de dépôt international: PCT/FR2004/001116
(87) Numéro de publication internationale: WO 2004/100924

(56) Documents cités:
- US-A- 4 380 534
- US-A- 4 761 407
- US-A1- 2002 010 164

## Description

La présente invention concerne un procédé de préparation d'une pommade contenant un principe actif ayant une concentration comprise entre 1 et 100 ppm, ledit procédé étant caractérisé par les étapes de préparation suivantes :
1. pesée d'un principe actif au sein d'un réceptacle tapissé de vaseline blanche ;
2. encapsulation du principe actif par de la vaseline blanche supplémentaire ;
3. introduction du réceptacle contenant le principe actif et la vaseline dans un mélangeur qui contient un mélange d'excipients chauffés à une température comprise entre 70 et 90°C ; et
4. diffusion du principe actif dans le mélangeur,
et en ce que le principe actif est le calcitriol.

Il est connu que la fabrication de préparations à faible concentration de principe actif entraîne des problèmes d'homogénéisation du principe actif dans ses excipients. En effet, des principes actifs microdosés vont rencontrer des problèmes d'hétérogénéité de dispersion lorsqu'ils sont mélangés à de grands volumes d'excipients.

Ces préparations nécessitent normalement le passage par une phase intermédiaire de dilutions permettant l'obtention de produits à faible concentration de principe actif.

Un problème supplémentaire surgit lorsque le principe actif appartient à une classe de produits toxiques. La manipulation devient alors non seulement longue mais aussi dangereuse, ce qui augmente de façon importante les risques d'accidents. S'ajoute aussi le problème du transport et de l'occultation du produit toxique, à l'abri de la lumière et des phénomènes d'oxydation.

Une solution à ce problème est un nouveau procédé, selon l'invention, de préparation d'une formulation contenant un principe actif, caractérisé en ce qu'il comprend les étapes de préparation suivantes :
1. pesée d'un principe actif au sein d'un réceptacle tapissé de vaseline blanche ;
2. encapsulation du principe actif par de la vaseline blanche supplémentaire ;
3. introduction du réceptacle contenant le principe actif et la vaseline dans un mélangeur qui contient un mélange d'excipients chauffés à une température comprise entre 70 et 90°C ; et
4. diffusion du principe actif dans le mélangeur.

Dans l'invention, le principe actif est le calcitriol.

Ces différentes étapes vont permettre un gain de temps et de qualité dans l'obtention d'un produit homogène.

En effet, la diffusion constante du principe actif à partir du réceptacle dans le mélangeur assurée par le procédé selon l'invention évite la dispersion hétérogène observée lors d'un ajout « en vrac » dans la cuve du mélangeur tel qu'il peut être réalisé antérieurement.

De plus, l'encapsulation du principe actif dans un cocon de vaseline permet de supprimer les étapes de dilution d'un principe actif et de circonscrire les risques de toxicité et d'accident de manipulations du principe actif après la pesée, notamment lors du transport de celui-ci vers la cuve.

Enfin, l'étanchéité créée par le procédé selon l'invention évite les problèmes d'oxydation et de dégradation par la lumière grâce à la protection apportée par la capsule de vaseline.

Cette invention, par la suppression des étapes intermédiaires de dilutions, va donc également réduire le temps de préparation.

Les pommades sont des préparations pour l'usage externe, destinées à une application directe sur la peau.

Quand le principe actif dans la pommade est un solide, il est saupoudré aussi finement que possible et incorporé dans la pommade par le principe de la dilution géométrique.

La dilution géométrique implique une série d'étapes de dilution. Elle commence par l'incorporation du principe actif dans une quantité d'excipient approximativement de la même taille.

Une deuxième quantité d'excipient approximativement égale au premier mélange réalisé est ajoutée et alors mélangée.

Ce processus par étapes de dilution est réalisé jusqu'à ce que la totalité de l'excipient soit utilisé pour obtenir la concentration en principe actif souhaitée.

Les différents produits entrant dans la fabrication de la pommade sont généralement mélangés ensemble par fusion sur un bain d'eau, suivi d'une agitation jusqu'à refroidissement du mélange.

Le principe actif étant incorporé à une étape appropriée.

C'est ce processus itératif qui est essentiel pour produire une pommade homogène.

Le brevet US 4,380,534 décrit une préparation d'un principe actif solide contenant une poudre de principe actif microdosé enrobée de cire.

Le brevet US 4,761,407 décrit des formulations orales instantanées sous forme solides (ISOF) comprenant une faible dose de principe actif.

La demande de brevet US 2002/0010164 décrit des compositions aqueuses comprenant une faible quantité de 1α, 25-dihydroxycholecalciferol ou calcitriol.

### Description Générale de l'invention:

Au sens le plus large, l'invention concerne un procédé tel que défini dans les revendications attachées.

L'invention concerne un procédé de préparation d'une formulation, de préférence une pommade, contenant un principe actif microdosé. Ledit procédé étant caractérisé par les étapes de préparation suivantes :
1. pesée d'un principe actif au sein d'un réceptacle tapissé de vaseline ;
2. encapsulation du principe actif par de la vaseline supplémentaire ;
3. introduction du réceptacle contenant le principe actif et la vaseline dans un mélangeur ; et
4. diffusion du principe actif dans le mélangeur.

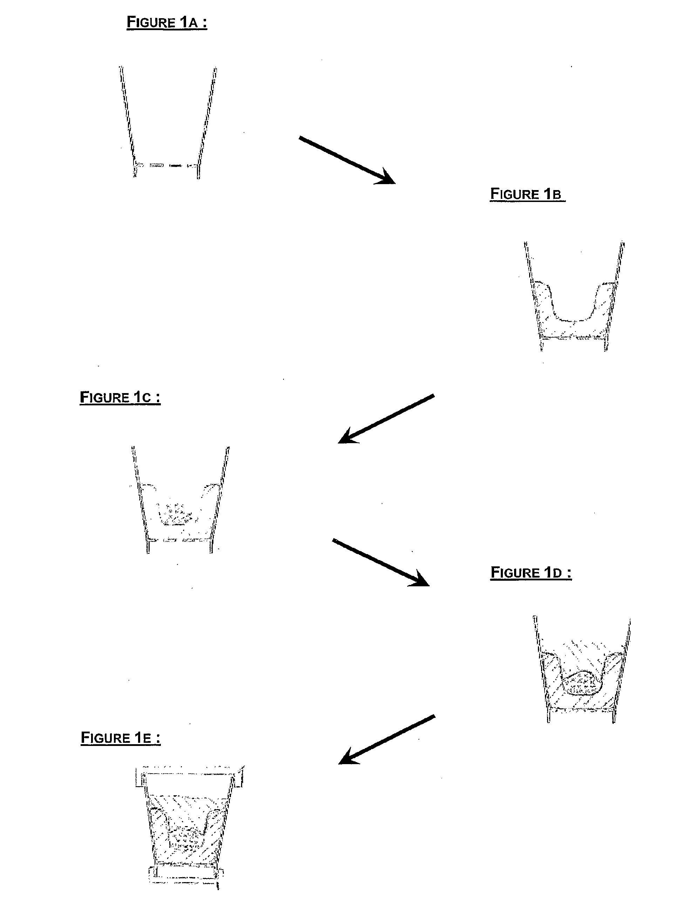

De façon particulière, la préparation d'une formulation liquide homogène contenant un principe actif microdosé se fait comme suit :
- Lors de l'étape 1, le réceptacle va être dans un premier temps taré (voir figure 1a) avant d'être façonné dans le fond par une couche de vaseline blanche (voir figure 1b).
   Il faudra alors effectuer une pesée permettant de déterminer le poids de vaseline ajoutée. Ensuite, on va introduire le principe actif dans le réceptacle comme indiqué en figure 1c. On pèsera alors à nouveau le réceptacle pour déterminer la quantité exacte de principe actif ajoutée.
- Pendant l'étape 2, un bouchon de vaseline blanche sera façonné (voir figure 1d) permettant d'envelopper le principe actif de vaseline. Cette étape délicate doit être faite de telle sorte qu'il n'y ait pas de formation de poches d'air qui nuiraient à la formation du " cocon " de vaseline blanche.

Pour finir la préparation du réceptacle, ce dernier va être fermé par deux bouchons rigides (voir figure 1e).

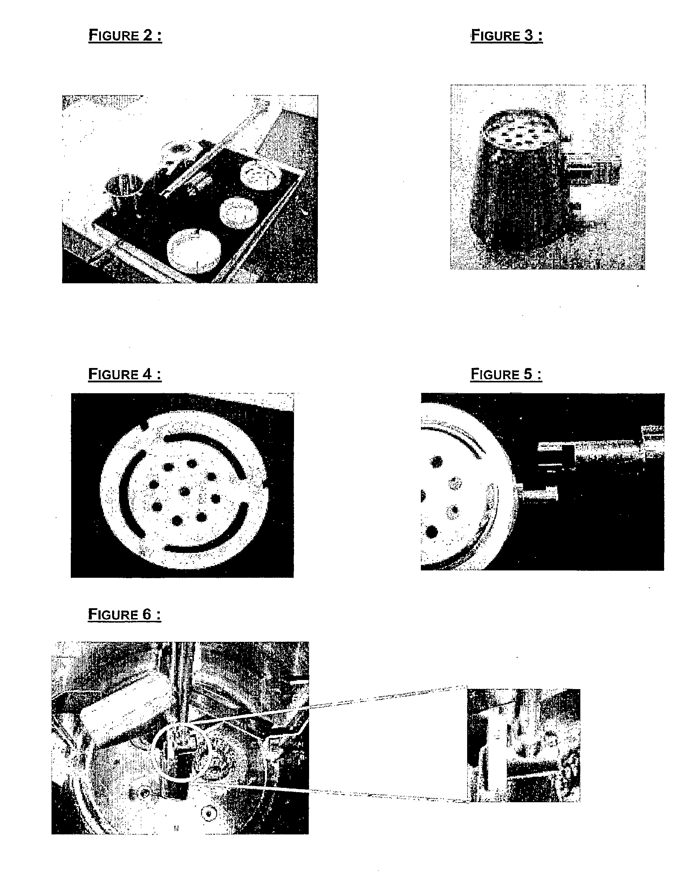

Sous cette forme fermée du réceptacle, le principe actif encapsulé par de la vaseline blanche va pouvoir être transporté vers son lieu de façonnage à l'abri de la lumière et des phénomènes d'oxydation.
- Pour la réalisation de l'étape 3 suivante, le petit bouchon du réceptacle sera enlevé et le gros bouchon du réceptacle sera remplacé par un bouchon perforé (voir figures 3 et 4).
   De telle manière, le réceptacle pourra être introduit dans une cuve (voir figure 6), cette cuve et son contenu d'excipients sont suffisamment chauffés pour que le mélange d'excipients qu'elle contient soit sous forme liquide.
- Au cours de l'étape 4, la vaseline solide encapsulant le principe actif va fondre au contact du mélange d'excipients chauffés. Ainsi, elle pourra diffuser et se répartir de façon homogène parmi les autres excipients contenus dans la cuve du mélangeur, entraînant avec elle la dissolution du principe actif.

Cette répartition homogène est rendue possible par différents éléments, tout d'abord par la température des excipients de la cuve, comprise entre 70 et 90°C, qui permet donc de faire fondre la vaseline contenue dans le réceptacle diffuseur contenant le principe actif. La répartition homogène est aussi rendue possible ensuite, par les grilles des extrémités du réceptacle qui ne laissent diffuser le principe actif que de manière constante et en faible quantité. Les grilles empêchent donc la formation de grosses masses de vaseline semi-solide qui entraîneraient une grande partie du principe actif, aboutissant à une mauvaise répartition et donc une mauvaise homogénéisation du produit final.

Par principe actif microdosé, on entend une concentration en principe actif comprise entre 1 et 100 ppm, par exemple entre 1 et 50 ppm, en particulier entre 1 et 10 ppm et de préférence entre 1 et 5 ppm.

Dans l'invention, le principe actif utilisé dans le procédé selon l'invention est le calcitriol, en particulier, on préférera utiliser du calcitriol à une concentration finale choisie parmi 3 ppm, 6 ppm et 9 ppm, en particulier à une concentration de 3 ppm.

Il est bien entendu possible, par le procédé décrit, de préparer des compositions comprenant plusieurs agents actifs dont un ou plusieurs sont microdosés.

Par pesée, on entend une action de tarer ou de façon plus générale, une manière de déterminer le poids, la masse d'un produit.

De façon avantageuse, pour éviter tout risque de contamination avec un principe actif toxique, on effectuera l'étape de pesée sous hotte à flux laminaire verticale ou sous isolateur.

De façon avantageuse, pour éviter toute imprécision, cette étape de pesée se fera sur balance d'une précision au 1/100 de mg.

Par réceptacle, on entend tout volume, récipient, réservoir permettant l'introduction d'un principe actif. L'invention ici se rapporte plus particulièrement à un réceptacle de type tronc de cône.

De façon avantageuse, pour faciliter la diffusion, on utilise un tronc de cône qui, de par sa forme, aura la particularité de faciliter et d'augmenter la vitesse du fluide. La vitesse va être plus modérée dans la partie large, ce qui va permettre une meilleure dissolution du principe actif.

Par réceptacle métallique, on entend préférentiellement réceptacle en inox.

Dans un mode de réalisation particulier du présent procédé, le réceptacle permettant la diffusion optimale du principe actif calcitriol pour la préparation du produit Silkis comprend les pièces suivantes (voir figure 2) :
1- un tronc de cône métallique (voir figure 3, en inox, qui sera recouvert de vaseline lors de la préparation de la formulation ;
2- deux bouchons pleins et un gros bouchon perforé (voir figures 2 et 4) en téflon blanc. Ces bouchons à système de baïonnette viennent s'encliqueter sur le réceptacle ;
3- la forme du gros bouchon perforé (voir figure 4) et de la grille de la petite extrémité du réceptacle (voir figure 3) vont permettre une diffusion de la vaseline fondue, lorsque celle-ci sera en contact avec un excipient chaud, empêchant la formation de grumeaux.

La fixation du réceptacle sur une tige fixée en hauteur (selon la configuration de la cuve) se fait par emboîtement comme schématisé en figure 5. Le réceptacle pourra sous cette forme fixée en hauteur tourner pour permettre une répartition homogène du principe actif dans la cuve (voir figure 6).

Par bouchon rigide, on entend préférentiellement en téflon ou en inox.

Par vaseline, on entend toute substance grasse dérivée du pétrole entrant dans la composition de pommades. L'invention se rapporte ici plus particulièrement à une vaseline répondant aux spécifications de la pharmacopée européenne et américaine « Pharmeuropa » et « USP », de viscosité comprise entre 130 000 et 550 000 Cps.

De façon avantageuse, on utilise une vaseline permettant une limitation maximale des phénomènes d'exudation de l'huile de paraffine.

De façon avantageuse, pour permettre la formation d'un ensemble hermétique, cette opération sera effectuée sous vide, ce qui permet la formation d'un ensemble hermétique pouvant être conserver pendant une période plus longue.

Par mélange d'excipients, on entend tout mélange contenant deux excipients ou plus, par exemple un mélange de vaseline et de paraffine, en particulier un mélange de 50 à 60% de vaseline blanche et de 40 à 50% de paraffine.

Par chauffée, on entend toute production de chaleur permettant d'élever la température des excipients de telle sorte que lesdits excipients soient sous forme liquide.

De façon avantageuse, pour permettre une homogénéisation optimale, il est important de respecter une température et un temps d'homogénéisation définis. Par exemple, dans un mode de réalisation préféré de l'invention, la température est comprise entre 70 et 90°C et le temps d'homogénéisation entre 2h et 7h, en particulier entre 3h30 et 5h30.

### Détails des figures :

**FIGURE 1A****:** Tarage du réceptacle
**FIGURE 1B** **:** Façonnage et pesée de la couche de vaseline blanche
**FIGURE 1C** **:** Dépôt et pesée du Calcitriol
**FIGURE 1D** **:** Façonnage du bouchon de vaseline
**FIGURE 1E** **:** Fermeture du réceptacle par deux bouchons rigides
**FIGURE 2** **:** Vue de haut des différentes pièces du réceptacle
**FIGURE 3** **:** Tronc de cône du réceptacle (vue latérale)
**FIGURE 4** **:** Bouchon perforé à baïonnette (vue de haut)
**FIGURE 5** **:** Emboîtement du réceptacle sur la tige (vue de haut)
**FIGURE 6** **:** Réceptacle fixé dans la cuve (vue de haut)

### Exemple 1a : Réalisation de la pesée du Calcitriol dans le réceptacle.

Dans un premier temps, il faut préparer un pot et un tube de vaseline qui serviront pour l'étape de pesée du Calcitriol.

La pesée du pot et du tube est effectuée de la façon suivante :
- Mettre le pot et son couvercle sur la balance 8 kg ;
- Tarer la balance ;
- Prélever environ 50g de vaseline et l'introduire dans le pot ;
- Refermer le pot et remettre la balance à zéro ;
- Mettre le tube sur la balance ;
- Tarer la balance ;
- Prélever environ 50g de vaseline et l'introduire dans le tube ;et
- Fermer le tube à l'aide de la plieuse.

Après cette phase de préparation de la vaseline, le tronc de cône est préparé avec la vaseline comme suit :
Tout d'abord, il faut mettre le tronc de cône en inox muni de son bouchon inférieur et son bouchon supérieur pleins sur le plateau de la balance, on tare alors la balance avant de remplir le réceptacle de vaseline à l'aide d'une spatule. Le tronc de cône doit être au 2/3 plein et posséder en son centre un puit où sera introduit le calcitriol. On notera alors la masse de vaseline ajoutée avant d'introduire ledit tronc de cône de vaseline dans l'isolateur.

L'ampoule de calcitriol est introduite au préalable dans l'isolateur afin qu'elle soit à la même température que l'enceinte au moment de la pesée.

On va ensuite peser par différence le calcitriol introduit dans le tronc de cône. Cette étape d'introduction du calcitriol se fait par versement progressif, de telle sorte qu'on évite la dissémination de la poudre.

Suite à cette étape de pesée du principe actif, on va recouvrir le puit contenant le calcitriol par de la vaseline contenue dans le tube.

Le tronc de cône ainsi constitué sera recouvert par les deux bouchons pleins à baïonnette et pourra être transporté à l'abri de tout risque de toxicité vers son lieu de façonnage.

### Exemple 1b : Fabrication du produit Silkis 3 ppm.

1a) la cuve de fabrication est remplie avec de la vaseline blanche, afin qu'elle représente au final 56.2487% de la formulation. La présente vaseline est d'abord préfondue à une température permettant son passage à un état liquide, puis chauffée sous azote et sous agitation dans des conditions de température permettant de maintenir cette vaseline à l'état liquide et homogène.
1b) En parallèle, un fondoir est rempli avec de la Paraffine liquide, afin qu'elle représente au final 43.75% de la formulation. On chauffe ensuite sous azote et sous agitation à une température équivalente à celle utilisée pour la vaseline dans l'étape 1a).
1c) On va également préparer sous isolateur le principe actif pendant cette première étape. Pour se faire, on va peser le calcitriol dans un cocon de vaseline, sous lumière inactinique et azote, de telle sorte que le Calcitriol représente 0.0003% de la formulation finale.
2) Après préparation de ces trois composés, dans un premier temps la paraffine chaude est introduite dans la cuve contenant la vaseline.
3) Puis est ajouté à ce mélange d'excipients du D.L alpha tocophérol en concentration telle qu'il représente au final 0.001% de la formulation
4) Ce mélange des trois excipients va alors subir un dégazage et un maintien sous azote.
5) S'en suit une phase d'homogénéisation pendant laquelle les agitations sont maintenues sous vide.
6) Après cette période d'homogénéisation, on va, sous lumière inactinique, introduire le tronc de cône contenant le Calcitriol dans la cuve contenant les trois excipients.
7) A la suite de cette étape, on va effectuer un dégazage et réinertage à l'azote, sous vide.
8) On va par la suite soumettre cet ensemble le temps nécessaire pour une homogénéisation sous vide.
9) Après cette dernière phase d'homogénéisation, on va laisser la formulation refroidir sous vide afin d'éviter toute incorporation d'air dans le mélange.
10) La dernière étape sera le conditionnement primaire à l'état liquide sous azote, sous agitation lente et sous lumière inactinique.

## Revendications

1. Procédé de préparation d'une pommade contenant un principe actif ayant une concentration comprise entre 1 et 100 ppm, ledit procédé étant **caractérisé par** les étapes de préparation suivantes :
1. pesée d'un principe actif au sein d'un réceptacle tapissé de vaseline blanche ;
2. encapsulation du principe actif par de la vaseline blanche supplémentaire ;
3. introduction du réceptacle contenant le principe actif et la vaseline dans un mélangeur qui contient un mélange d'excipients chauffés à une température comprise entre 70 et 90°C ; et
4. diffusion du principe actif dans le mélangeur,
et en ce que le principe actif est le calcitriol.

2. Procédé selon la revendication 1, **caractérisé par le fait que**, pour éviter tout risque de contamination avec un principe actif toxique, l'étape de pesée est effectuée sous hotte à flux laminaire verticale ou sous isolateur.

3. Procédé selon la revendication 1 ou 2, **caractérisé par le fait que** le volume permettant l'introduction d'un principe actif est un réceptacle de type tronc de cône.

4. Procédé selon l'une quelconque des revendications 1 à 3, **caractérisé en ce que** ladite vaseline permet une limitation maximale des phénomènes d'exsudation de l'huile de paraffine.

5. Procédé selon les revendications 1 à 4, **caractérisé en ce que** le mélange d'excipients est un mélange de vaseline et de paraffine.

6. Procédé selon la revendication 4 ou 5, **caractérisé en ce que** le mélange d'excipients est constitué de 50 à 60% de vaseline blanche et de 40 à 50% de Paraffine.

7. Procédé selon la revendication 1, **caractérisé en ce que** le calcitriol est à une concentration finale de 3 ppm.

## Patentansprüche

1. Verfahren zur Herstellung einer Formulierung, enthaltend einen Wirkstoff mit einer Konzentration zwischen 1 und 100 ppm, wobei besagtes Verfahren durch die nachfolgenden Herstellungsschritte:
1. Abwiegen eines Wirkstoffs in einem mit weißer Vaseline ausgekleideten Gefäß;
2. Einschließen des Wirkstoffs mit zusätzlicher weißer Vaseline;
3. Einführen des Gefäßes, das den Wirkstoff und die Vaseline enthält, in einen Mischer, der ein Gemisch von Arzneimittelträgern enthält, die auf eine Temperatur zwischen 70 und 90°C erwärmt sind; und
4. Diffusion des Wirkstoffs im Mischer,
und **dadurch gekennzeichnet ist, dass** der Wirkstoff Calcitriol ist.

2. Verfahren gemäß Anspruch 1, durch die Tatsache gekennzeichnet, dass zur Vermeidung jeglichen Kontaminationsrisikos mit einem toxischen Wirkstoff der Schritt des Abwiegens in einer Sterilbank oder in einem Isolator durchgeführt wird.

3. Verfahren gemäß Anspruch 1 oder 2, durch die Tatsache gekennzeichnet, dass das Volumen, das die Zugabe eines Wirkstoffs erlaubt, ein kegelstumpfförmiges Gefäß ist.

4. Verfahren gemäß einem der Ansprüche 1 bis 3, **dadurch gekennzeichnet, dass** besagte Vaseline eine maximale Begrenzung der Exsudation des Paraffinöls erlaubt.

5. Verfahren gemäß Ansprüchen 1 bis 4, **dadurch gekennzeichnet, dass** das Gemisch der Arzneimittelträger ein Gemisch aus Vaseline und Paraffin ist.

6. Verfahren gemäß Anspruch 4 oder 5, **dadurch gekennzeichnet, dass** das Gemisch der Arzneimittelträger aus 50 bis 60% weißer Vaseline und 40 bis 50% Paraffin besteht.

7. Verfahren gemäß Anspruch 1, **dadurch gekennzeichnet, dass** Calcitriol mit einer Endkonzentration von 3 ppm vorliegt.

## Claims

1. Process of preparation of an ointment containing an active principle having a concentration comprised between 1 and 100 ppm, said process being **characterized by** the following steps of preparation:
1. weighting of an active principle in a receptacle lined with a white petroleum jelly;
2. encapsulating of the active principle with additional white petroleum jelly;
3. introducing the receptacle containing the active principle and petroleum jelly into a mixer that contains a mixture of excipients heated at a temperature comprised between 70 and 90°C; and
4. diffusing the active principle in the mixer,
and **characterized in that** the active principle is calcitriol.

2. Process according to claim 1, **characterized in that** weighting step is carried out in a fume cupboard with laminar flow or in a sealed enclosure, to avoid any risk of contamination with a toxic active principle.

3. Process according to claim 1 or 2, **characterized in that** the volume allowing the introduction of an active principle is a conic frustum receptacle.

4. Process according to anyone of claims 1 to 3, **characterized in that** said petroleum jelly allows maximal limitation of the paraffin oil exudation phenomena.

5. Process according to any one of claims 1 to 4, **characterized in that** the mixture of excipients is a mixture of petroleum jelly and paraffin.

6. Process according to claim 4 or 5, **characterized in that** the mixture of excipients is constituted from 50 to 60% of white petroleum jelly and from 40 to 50% of paraffin.

7. Process according to claim 1, **characterized in that** calcitriol is at a final concentration of 3 ppm.
